**Europäisches Patentamt**

**European Patent Office**

**Office européen des brevets**

(19)

(11) Veröffentlichungsnummer: **0 003 588 B1**

(12) **EUROPÄISCHE PATENTSCHRIFT**

(45) Veröffentlichungstag der Patentschrift:
25.11.81

(21) Anmeldenummer: **79100335.3**

(22) Anmeldetag: **05.02.79**

(51) Int. Cl.³: **H 03 G 3/34** // A61B5/04, H04B15/00

(54) **Verfahren und Schaltungsanordnung zur Unterdrückung von Störsignalen in einem Nutzsignal.**

(30) Priorität: **10.02.78 DE 2805681**

(43) Veröffentlichungstag der Anmeldung:
**22.08.79 Patentblatt 79/17**

(45) Bekanntmachung des Hinweises auf die Patenterteilung:
**25.11.81 Patentblatt 81/47**

(84) Benannte Vertragsstaaten:
**IT NL SE**

(56) Entgegenhaltungen:
**CH-A-524 290**
**DE-A-2 246 100**
**FR-A-2 028 935**
**US-A-3 947 636**
**US-A-3 950 694**
**US-A-4 076 969**

(73) Patentinhaber: **SIEMENS AKTIENGESELLSCHAFT Berlin und München, Postfach 22 02 61, D-8000 München 22 (DE)**

(72) Erfinder: **Maas, Michael, Schleifweg 7 b, D-8521 Uttenreuth (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

### Verfahren und Schaltungsanordnung zur Unterdrückung von Störsignalen in einem Nutzsignal

Die Erfindung bezieht sich auf ein Verfahren und eine Schaltungsanordnung zur Unterdrückung von Störsignalen in einem Nutzsignal, wobei die Störsignale als Gemisch aus schmalen Störimpulsspitzen hoher Amplitude und Störbrumm anfallen können und wobei mittels Störspitzen- und Störbrumm-Erkennungseinrichtungen das Vorhandensein von Störimpulsspitzen und/oder Störbrumm separat erkannt wird und mit dem Erkennen Unterdrückereinrichtungen zur Unterdrückung der Störimpulsspitzen und/oder des Störbrumms im Nutzsignal aktiviert werden und wobei der Störbrumm bei Vorhandensein speziell durch ein Störbrummfilter erkannt und durch gegensinnige Überlagerung mit dem gestörten Nutzsignal vom Nutzsignal eliminiert wird.

Insbesondere in der Medizintechnik, wo einem Patienten beispielsweise physiologische Signale abgenommen werden, werden in der Signalableitung häufig Störspannungen, wie Nullinienschwankungen, Störamplitudenspitzen, die aus Muskelzuckungen oder Muskelzittern bzw. künstlich zugeführten Reizimpulsen resultieren, und Netzfrequenzstörungen als höherfrequenter Störbrumm beobachtet. Speziell bei der Ableitung eines Elektrokardiogramms können dabei dem EKG-Signal zusätzlich zu einem Netzbrumm auch noch Schrittmacherimpulse eines Herzschrittmachers überlagert sein. Dies gilt insbesondere immer für solche Patienten, die zur Unterstützung ihrer Herztätigkeit einen Herzschrittmacher tragen. Schrittmacherimpulse im EKG sind nicht in jedem Fall unerwünschte Störspannung; da sie Träger der Information über eine erfolgte Stimulierung des Herzens sind, kann in besonderen Fällen der Wunsch bestehen, zwecks spezieller Auswertung EKG-Signale zusammen mit Herzschrittmacherimpulsen abzubilden. Im Normalfall, d. h. bei gewöhnlicher praktischer Anwendung, wie Herzfrequenzmessung, EKG-Registrierung etc., wirkt sich das Vorhandensein von Schrittmacherimpulsen jedoch erheblich störend aus; durch die Schrittmacherimpulse wird nämlich im allgemeinen der Informationsgehalt des EKG's so verändert, daß dessen Auswertung, sei es visuell durch das Auge oder automatisch durch eine geeignete Auswerteeinheit, erschwert oder gar unmöglich wird. In diesen Fällen muß die Spitzenstörspannung, also beispielsweise der Schrittmacherimpuls, im Nutzsignal unterdrückt werden.

Eine solche Störspitzenunterdrückung bereitet im allgemeinen keine Schwierigkeiten, sofern nicht gleichzeitig auch noch weitere Störkomponenten, wie beispielsweise der Netzbrumm, unterdrückt werden sollen. Eine Vorrichtung, die eine Unterdrückung von Störimpulsspitzen in einem Nutzsignal, d. h. speziell auch von Herzschrittmacherimpulsen in einem Elektrokardiogramm, erlaubt, ist aus der britischen Patentschrift 1 450 081 ( = DE-A-2 246 100) vorbekannt. Die Unterdrückung der Störimpulsamplituden geschieht jedoch dort ohne die gleichzeitige Unterdrückung einer Störbrummkomponente. Entsprechend erlaubt beispielsweise auch die Vorrichtung der DE-AS 2 558 126 oder jene der CH-PS 524 290 nur die Unterdrückung von Netzbrumm im EKG ohne Beisein und gleichzeitige Unterdrückung von Störspitzen, wie Schrittmacherimpulsen od. dgl. Eine Vorrichtung, die eine Unterdrückung sowohl von Störimpulsspitzen als auch Störbrumm in einer gemeinsamen Schaltungsanordnung ermöglicht, ist zwar bereits durch die US-PS 3 950 694 vorbekannt; gerade bei einer solchen Schaltungsanordnung sind jedoch Rückwirkungen der Austastung von Störamplitudenspitzen auf die Unterdrückung des Netzbrumms nicht von vornherein mit Sicherheit auszuschließen. So besteht Gefahr, daß während der Zeitdauer der Unterdrückung einer Störspitzenamplitude aufgrund der dabei auftretenden Unterbrechungen bzw. auch Sprüngen im Signalverlauf die Erkennungseinrichtung bzw. auch Unterdrückereinrichtung für den Störbrumm in unkontrollierbarer Weise beeinflußt wird. In umgekehrtem Sinne wirkt sich jedoch aber auch ein vom Störbrumm noch nicht oder nur ungenügend gereinigtes Nutzsignal ungünstig aus für die Erkennung von Störimpulsspitzen und deren Unterdrückung. Bei Herzschrittmacherimpulsen ergeben sich dann beispielsweise Schwierigkeiten bei der Kompensation insbesondere des Schrittmacherüberschwingers, da ein EKG mit Brummstörungen sich kaum in einen e-Funktionsverlauf umformen läßt oder ein solcher sich aus dem EKG ableiten läßt, der für die exakte Kompensation des Überschwingers erforderlich ist. Ungewöhnlich hohe Brummamplituden können darüber hinaus von der Störimpulsspitzen-Erkennungseinrichtung fälschlicherweise als Störimpulsspitzen gewertet werden, so daß es zu unnötigen und möglicherweise sogar Meßergebnis verfälschenden Signalunterdrückungen kommen kann.

Aufgabe der Erfindung ist es, ein Verfahren und eine Schaltungsanordnung der eingangs genannten Art anzugeben, das/die eine Unterdrückung solcher Störsignale erlaubt, die im Gemisch schlimmstenfalls sowohl schmale Störimüulsspitzen hoher Amplitude als auch Störbrumm aufweisen, wobei gegenseitige Beeinflussungen bei Erkennen und Unterdrücken des einen Störsignalanteils durch den jeweils anderen mit Sicherheit nicht auftreten.

Die Aufgabe wird mit einem Verfahren der eingangs genannten Art dadurch gelöst, daß ein anfallendes Nutzsignal mit möglicherweise überlagerten Störsignalen nur so lange einem speziell auch auf Eigenschwingbetrieb umschaltbaren Störbrummfilter zwecks Herausfilterung speziell des Störbrumms zugeführt wird, wie von der Erkennungseinrichtung für schmale Störimpulsspitzen hoher Amplitude keine solche Störimpulsspitzen erkannt werden, und daß auch nur in diesem Fall die herausgefilterte Störbrummkomponente im Originalverlauf dem Nutzsignal im Meßsignalweg vor der Erkennungseinrichtung für Störimpulsspitzen zwecks Kompensation des diesem überlagerten Störbrumms gegensinnig überlagert wird, daß jedoch in dem Fall, daß von der Erkennungseinrichtung eine Störimpulsspitze erkannt wird, das Störbrummfilter vom Eingangssignal abgeschaltet und gleichzeitig auf Eigenschwingbetrieb umgeschaltet wird, so daß es mit der Amplitude und Frequenz der vorhergehend selektierten Störbrummkomponente ohne Phasensprung weiterschwingt, wobei als Anfangswert für die Eigenschwingung des Störbrummfilters jener Signalwert dient, der im Augenblick des Erkennens einer Störimpulsspitze der unmittelbar vorhergehende Signalwert der Eingangsspannung ist und der einerseits in einem ausgangsseitigen Speicher als ausgangsseitiges Nutzsignal für die Zeitdauer einer Austastung der Störimpulsspitze gespeichert wird und am Ende der Austastung den Anfangswert für die Fortsetzung des Signalspannungsverlaufs bildet und der andererseits zur Bildung des Anfangswertes für die Eigenschwingung auch zum Eingang des Störbrummfilters rückgeführt wird, und daß die vom Störbrummfilter so künstlich erzeugte Schwingung schließlich wenigstens während der Zeitdauer der Unterdrückung der Störimpulsspitze dem Nutzsignal im Meßsignalweg vor der Erkennungseinrichtung zur Kompensation des Störbrumms überlagert wird, so daß der Erkennungseinrichtung immer von Störbrumm bereinigtes Nutzsignal angeboten wird.

Eine Schaltungsanordnung, die nach diesem Verfahren arbeitet und die einerseits eine Störspitzen-Erkennungseinrichtung sowie eine davon gesteuerte Austasteinrichtung für Störimpulsspitzen umfaßt und die andererseits auch ein Störbrummfilter beinhaltet, welches einen Eingang für das möglicherweise mit Störsignalen überlagerte Nutzsignal aufweist und welches mit einem Ausgang versehen ist, der zum Zwecke der gegensinnigen Überlagerung des Nutzsignals samt Störsignal mit dem vom Filter ausgefilterten Störbrumm am Meßsignal angeschaltet ist, ist erfindungsgemäß dadurch gekennzeichnet, daß der Eingang des Störbrummfilters über einen Ab- oder Umschalter vom Eingangssignal abschaltbar ist, welcher Schalter von einem Schaltausgang der Erkennungseinrichtung für Störimpulsspitzen bei Auftreten einer solchen Spitze aktivierbar ist, daß das Störbrummfilter einen Rückkreis aufweist, der einen Rückkoppelschalter umfaßt, der gegensinnig zum Ab- oder Umschalter geschaltet wird und der auch eine Mischstufe beinhaltet, der das Ausgangssignal des Störbrummfilters und ein Spannungswert zuführbar sind, der einem gespeicherten Ausgangsspannungswert des Nutzsignals entspricht, der im Augenblick des Erkennens einer Störimpulsspitze durch die Störimpulsspitzenerkennungseinrichtung in einer Speichereinheit gespeichert wurde, und daß der Erkennungseinrichtung für eine Störimpulsspitze eine Mischstufe für ein Nutzsignal mit möglicherweise überlagertem Störbrumm und überlagerter Störimpulsspitze und für das Ausgangssignal des Störbrummfilters vorgeschaltet ist.

Gemäß der Erfindung wird dann, wenn einem Nutzsignal lediglich ein Störbrumm überlagert ist, dieser Störbrumm ständig vom Störbrummfilter herausgefiltert. Durch die gegensinnige Überlagerung mit dem ungefilterten Nutzsignal wird der Störbrumm eliminiert. Die Eliminierung des Störbrumms erfolgt bereits am Eingangssignal, so daß also der Erkennungseinrichtung für Störimpulsspitzen immer ein vom Störbrumm bereinigtes Nutzsignal angeboten wird. Ein Einfluß des Störbrumms auf Erkennen und Unterdrückung von Störimpulsspitzen ist somit nicht mehr gegeben. Tritt jedoch eine Störimpulsspitze auf, so wird unmittelbar mit dem Erkennen dieser Störimpulsspitze durch die Erkennungseinrichtung ein Abschaltsignal für das Störbrummfilter gegeben. Die Störimpulsspitze kann somit nicht zum Störbrummfilter gelangen, wodurch dieses in der Impulsantwort auch nicht in unzulässiger Weise durch die Störimpulsspitze beeinflußt werden kann. Obgleich jedoch das Störbrummfilter eingangsseitig vom Nutzsignal mit möglicherweise überlagertem Störbrumm abgeschaltet ist, so daß eigentlich kein Störbrumm mehr selektiert wird, so ergibt sich dennoch in der Nutzsignalleitung eine Kompensation des Störbrumms. Dies wird erreicht, weil das Störbrummfilter jetzt im Eigenschwingbetrieb die vorher vorhandene Störbrummkomponente nach Amplitude und Frequenz simuliert. War demnach vor Abschaltung des Störbrummfilters im Eingangssignal neben dem eigentlichen Nutzsignal auch noch Störbrumm vorhanden, so schwingt das Filter nach Abschaltung mit dieser Störbrummkomponente ohne Phasensprung weiter. Die entsprechende Störbrummkomponente im Nutzsignal kann somit auch weiterhin kompensiert werden. Enthält jedoch das Nutzsignal überhaupt keine oder nur wenig Brummstörung, so ist das Ausgangssignal des Störbrummfilters mangels ausreichend ausfilterbarer Störbrummkomponente paktisch gleich Null. Es wird dem Ursprungssignal demnach also keine Störbrummkomponente seitens des Störbrummfilters überlagert, so daß das von Störsignalen bereits freie Nutzsignal, sofern auch keine Störimpulsspitzen vorhanden sind, ungehindert zur Weiterverarbeitung und/oder Anzeige gelangen kann.

Weitere Vorteile und Einzelheiten der Erfindung ergeben sich aus der nachfolgenden Beschreibung eines Ausführungsbeispiels an hand der Zeichnung in Verbindung mit den Unteransprüchen.

Es zeigt

Fig. 1 die Darstellung eines zeitgedehnten EKG-Signals, dem Netzfrequenzstörungen und Herzschrittmacherimpulse überlagert sind,

Fig. 2 ein Ausführungsbeispiel der Erfindung im Prinzipschaltbild,

Fig. 3 die wesentlichsten Spannungsverläufe im Prinzipschaltbild der Fig. 2,

Fig. 4 eine Schaltungsanordnung gemäß dem Prinzipschaltbild der Fig. 2 in ausführlicherer Darstellung.

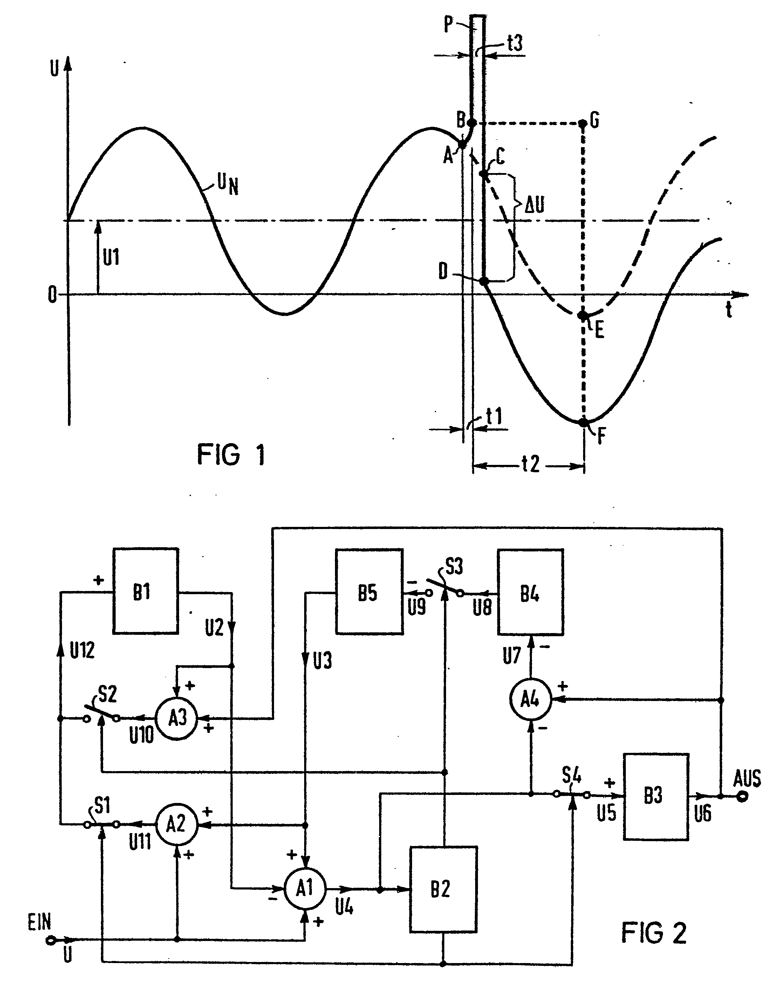

Die Fig. 1 zeigt in stark zeitgedehntem Maßstab ein Teilstück eines EKG-Signalverlaufs, wobei dem EKG-Signal U1 sowohl eine netzfrequente Störspannung $U_N$ als auch ein Herzschrittmacherimpuls P überlagert sind. Bei Punkt A beginnt der etwa 0,2 bis 1 ms (t3) breite Stimulationsimpuls des Herzschrittmachers. Seine Flanken sind etwas verschliffen (z. B. durch die Eigenschaften des EKG-Verstärkers bedingt). Deshalb wird er erst verspätet (t1) bei Punkt B erkannt. Die Amplitude des Simulationsimpulses ist so groß, daß der EKG-Verstärker in die Begrenzung geht. Während dieser Zeit enthält das EKG keine zusätzliche Information, d. h. über die wirkliche Größe des Stimulationsimpulses, die Amplitude der Herzaktionsspannung oder die überlagerte Netzfrequenzspannung ist keine Aussage möglich. Es können hier allenfalls Vermutungen angestellt werden (Extrapolation entsprechend der Vorgeschichte). Am Ende des Simulationsimpulses setzt die Spannung U(t) nicht bei Punkt C ihren Verlauf fort, wie sie es ohne Stimulationsimpuls getan hätte. Die Fortsetzung des Spannungsverlaufs erfolgt vielmehr bei Punkt D, der gegenüber dem Punkt C um die Spannung $\Delta U$ verschoben ist. Die Spannungsdifferenz $\Delta U$ ergibt sich aufgrund Überschwingens des Herzschrittmacherimpulses; dieser Überschwinger klingt näherungsweise nach einer e-Funktion ab (Zeitkonstante je nach Art des verwendeten Herzschrittmachers etwa 60 bis 250 ms).

Aus dem Spannungsverlauf U(t) der Fig. 1 ist zu erkennen, daß speziell dem Filter zur Netzfrequenzunterdrückung während der Phase t2 der Austastung eines Herzschrittmacherimpulses weder das (durchgezogene) Signal B, C, D, F noch das (punktierte) Signal B, G, E, F angeboten werden darf. In beiden Fällen würde durch diese Spannungsverläufe die Filterantwort in unzulässiger Weise beeinflußt. Zur Vermeidung dieses Nachteils muß also dem Filter ein Zeitverlauf der Spannung angeboten werden, der weitgehend jenem Verlauf entspricht, den die Spannung angenommen hätte, wenn der Schrittmacherimpuls nicht aufgetreten wäre. Dies ist der (strichlinierte) Spannungsverlauf A, C, E. Darüber hinaus darf anschließend am Eingang des Netzfrequenzfilters aber auch kein Spannungssprung von E nach F stattfinden, weil dieser Spannungssprung ebenfalls die Filterantwort verfälschen würde. Der weitere Spannungsverlauf im Anschluß an Punkt E soll vielmehr der um das Überschwingen des Schrittmacherimpulses verminderten Eingangsspannung entsprechen.

Eine weitere Schwierigkeit besteht darin, daß zur Unterdrückung des Schrittmacher-Überschwingers der Spannungswert am Ende der Austastphase gespeichert werden muß, damit er anschließend vom Eingangssignal subtrahiert werden kann. Auch dazu muß der Abstand E, F bekannt sein, wobei der Punkt E jedoch nur mittels Extrapolation vom Punkt A aus gefunden werden kann. Die Phase t2 der Austastung eines Schrittmacherimpulses soll einerseits lang genug sein, damit Störungen, die der Stimulationsimpuls verursacht hat, abgeklungen sind; andererseits soll sie aber auch möglichst kurz sein, damit die Extrapolation A, C, E mit großer Wahrscheinlichkeit gerade jenem Verlauf entspricht, den das Signal ohne überlagerten Schrittmacherimpuls nimmt. Als Kompromiß wird eine Zeitspanne von etwa t2 = 9 ms gewählt.

Im Prinzipschaltbild der Fig. 2 sind die Schaltungselemente für ein selektives Netzfrequenzfilter zu einer Baugruppe B1, jene für die Schaltung zur Erkennung eines Herzschrittmacherimpulses zur Baueinheit B2, jene für eine Verzögerungsleitung mit Speicher zur Baugruppe B3, die Schaltungselemente für einen Komparator zu einer Baugruppe B4 und schließlich die Schaltungselemente für ein Zeitkonstantglied zu einer Baugruppe B5 zusammengefaßt. Einzelne Baugruppen sind untereinander durch Analogschalter S1, S2, S3 und S4 verbindbar. Die Baugruppen A1, A2, A3 und A4 sind Mischstufen für anfallende Spannungssignale.

Die prinzipielle Arbeitsweise des Schaltbildes der Fig. 2 ist die, daß innerhalb der Schaltungsanordnung das am Signaleingang EIN anfallende Signalgemisch auf einzelne interessierende Signalanteile selektiert wird. Durch geschickte Überlagerung einzelner Spannungsanteile werden dann Hilfssignale gewonnen, die weitgehend den Anforderungen der naturgetreuen Simulation ungestörter Signale entsprechen. Die Schalter S1 bis S4 sorgen dafür, daß die Hilfssignale dem jeweiligen Augenblickszustand der Unterdrückung eines Herzschrittmacherimpulses entsprechend in den Mischstufen A1 bis A4 gemischt werden.

Die Arbeitsweise des Prinzipschaltbildes der Fig. 2 soll nun anhand der wesentlichen vier Fälle
— ungestörtes EKG
— EKG mit Netzfrequenzstörung
— EKG mit Schrittmacherimpuls
— EKG mit Netzfrequenzstörung und Schrittmacherimpuls
beschrieben werden.


a) Ungestörtes EKG

Das ungestörte Eingangssignal U = U1 gelangt auf die Mischstufen A1 und A2. Die Spannung U3, die ein Charakteristikum für das Vorliegen eines Schrittmacherimpulses ist, ist Null, weil ein Schrittmacherimpuls nicht vorhanden ist. Über die Mischstufe A2 und den geschlossenen Schalter S1 gelangt das Eingangssignal $U = U1$ als Signal $U11 = U12$ auf das Netzfrequenzfilter B1. Da das EKG-Signal ungestört sein soll, ist aber auch der netzfrequente Anteil $U_N$ im EKG-Signal gleich Null oder zumindest sehr klein. Es ergibt sich also, wenn überhaupt, nur ein Ausgangssignal U2, des selektiven Netzfrequenzfilters B1 nahe Null. An die Mischstufe A1 gelangt also praktisch wieder nur der störungsfreie EKG-Signalanteil U1, so daß das Ausgangssignal U4 der A1 dem Ausgangssignal U1 entspricht. Dieses Signal gelangt über den ebenfalls geschlossenen Schalter S4 als Signal U5 auf die Verzögerungsleitung B3. Das Signal wird hier verzögert und im Frequenzbereich, der oberhalb des für das ungestörte EKG interessanten Bereichs liegt, beschnitten. Als U6 gelangt das nur sehr geringfügig geänderte Eingangssignal $U = U1$ an den Ausgang AUS des Prinzipschaltbilds der Fig. 2. An einem (nicht dargestellten) Anzeige- oder Registriergerät, z. B. EKG-Schreiber oder Oszilloskop, kann dann das Ausgangssignal U6 angezeigt bzw. registriert werden. Zusätzlich oder alternativ hierzu kann jedoch am Ausgang AUS auch eine Weiterverarbeitungseinrichtung für die EKG-Signale vorhanden sein. Hierbei kann es sich beispielsweise um eine Herzfrequenzmeßeinrichtung handeln. Ebensogut kann es aber auch eine solche Einrichtung sein, die neben oder anstelle von normalen QRS-Komplexen im EKG solche mit extrasystolischem Charakter erfaßt und anzeigt.

### b) EKG-Signal mit Netzfrequenzstörungen

Das Eingangssignal $U = U1 + U_N$ gelangt wie unter Punkt a) auf die Mischstufen A1 und A2. Da Schrittmacherimpulse nicht vorhanden sind, ist U3 wieder Null. U11 am Ausgang der Mischstufe A2 entspricht dem gestörten Eingangssignal $U = U1 + U_N$. Dieses Signal gelangt über den wiederum geschlossenen Schalter S1 als U12 auf das Netzfrequenzfilter B1. Das Netzfrequenzfilter B1 läßt vom Gesamtsignal U12 lediglich den Netzanteil hindurch. Es fällt somit eine Ausgangsspannung U2 an, die nach Amplitude und Phase der Netzfrequenzstörung im EKG-Signal entspricht. In der Mischstufe A1 wird dieses Netzsignal $U2 = U_N$ vom Eingangssignal $U = U1 + U_N$ subtrahiert. Das Ausgangssignal U 4 dieser Mischstufe A 1 ist also das von Netzfrequenzstörungen befreite EKG-Signal U1. Der weitere Verlauf entspricht jenem unter Punkt a) für das ungestörte EKG.

### c) EKG mit Schrittmacherimpulsen eines Herzschrittmachers

Solange kein Stimulationsimpuls im EKG-Signal auftritt, wird das Signal entsprechend a) ausgewertet. Tritt jedoch ein Schrittmacherimpuls auf, so wird er von der Impulserkennungseinrichtung B2 erkannt. Die Erkennungseinrichtung B2 erzeugt daraufhin an ihren Steuerausgängen zu den Schaltern S1 bis S4 einen Umschaltimpuls, der zur Umschaltung der Schalter S1 bis S4 aus dem gezeichneten in den jeweils anderen Schaltzustand führt. Die Dauer des Umschaltimpulses legt dabei die Austastzeit (t2) für den Schrittmacherimpuls fest.

Da der Schalter S4 geöffnet wird, bleibt die Ausgangsspannung U6 auf dem vorhergehenden Spannungswert stehen. Dieser Spannungswert, der dem Spannungswert im Punkt A gemäß Fig. 1 entspricht, wird im Speicher der Baueinheit B3 abgespeichert.

Der Schalter S1 öffnet beim Auftreten eines Schaltimpulses der Impulserkennungseinrichtung B2. Das Eingangssignal wird somit vom Eingang des selektiven Netzfrequenzfilters B1 abgeschaltet. auf diese Weise wird also der Schrittmacherimpuls vom Eingang des selektiven Netzfrequenzfilters B1 ferngehalten. Es schließt jedoch der Schalter S2. Damit wird jedoch über eine Rückkoppelleitung vom Ausgang der Baueinheit B3 über die Mischstufe A3 und den geschlossenen Schalter S2 der gespeicherte Spannungswert auf das selektive Netzfrequenzfilter B1 gegeben. Es wird somit im selektiven Netzfrequenzfilter der dem Pacemakerimpuls unmittelbar vorhergehende Spannungspegel der Eingangsspannung U simuliert.

Der Schalter S3 schließt. Hierdurch schließt sich auch eine Regelschleife S3, U9, B5, U3, A1, U4, A4, U7, B4 und U8. Damit wird in der ein Zeitkonstanten-Glied bildenden Baugruppe B5 eine Ausgangsspannung U3 hervorgerufen, die zusammen mit der Eingangsspannung U in der Mischstufe A1 eine Spannung erzeugt, die betragsmäßig dem gespeicherten Spannungswert U6 entspricht, zu diesem jedoch entgegengesetzt polarisiert ist. U3 ist also exakt der Negativwert der Eingangsspannungsänderung während der Phase der Austastung eines Schrittmacherimpulses. Das Zeitkonstanten-Glied B5 weist nun eine solche Zeitkonstante auf, daß U3 sich mit dem Schließen des Schalters S3 zwar schnell auf seinen Anfangsspannungswert einstellt; bei am Ende der Austastzeit wieder geöffnetem Schalter S3 wird die Spannung U3 jedoch langsam nach einer e-Funktion zu Null geführt. Die langsame Zeitkonstante ist dabei auf die Zeitkonstante des Abklingens des Überschwingers des Herzschrittmacherimpulses einstellbar. Mit dem Schließen des Schalters S3 stellt sich also die Spannung U3 auf den Spitzenwert des Überschwingers des Schrittmacherimpulses mit entgegengesetzter Polarität ein. Anschließend klingt mit dem Wiederöffnen des Schalters S3 die Spannung U3 von diesem entgegengesetzten Spitzenwert mit der Zeitkonstanten des Überschwingers auf Null ab. Die Überlagerung des Ursprungssignals U mit diesem Signalverlauf U3 in den Mischstufen A1 und A2 bewirkt die Kompensation des Überschwingers des Herzschrittmacherimpulses im Eingangssignal. Da mit dem Ende des Austastimpulses der Schrittmacherimpuls-Erkennungseinrichtung B2 auch der Schalter S1 wieder geschlossen bzw. der Schalter S2 jetzt geöffnet ist, wird dem Netzfrequenzfilter B1 ein von Überschwingern des Schrittmacherimpulses befreites Signal U 12 zugeleitet. In entsprechender Weise wird jedoch auch am Ausgang der Mischstufe A 1 ein von Überschwingern bereinigtes Signal U 4 erzeugt, das über den jetzt wieder geschlossenen Schalter S 4 zur Verzögerungsleitung und von dort zum Signalausgang AUS gelangt.

### d) EKG mit Netzfrequenzstörung und Schrittmacherimpulsen eines Herzschrittmachers

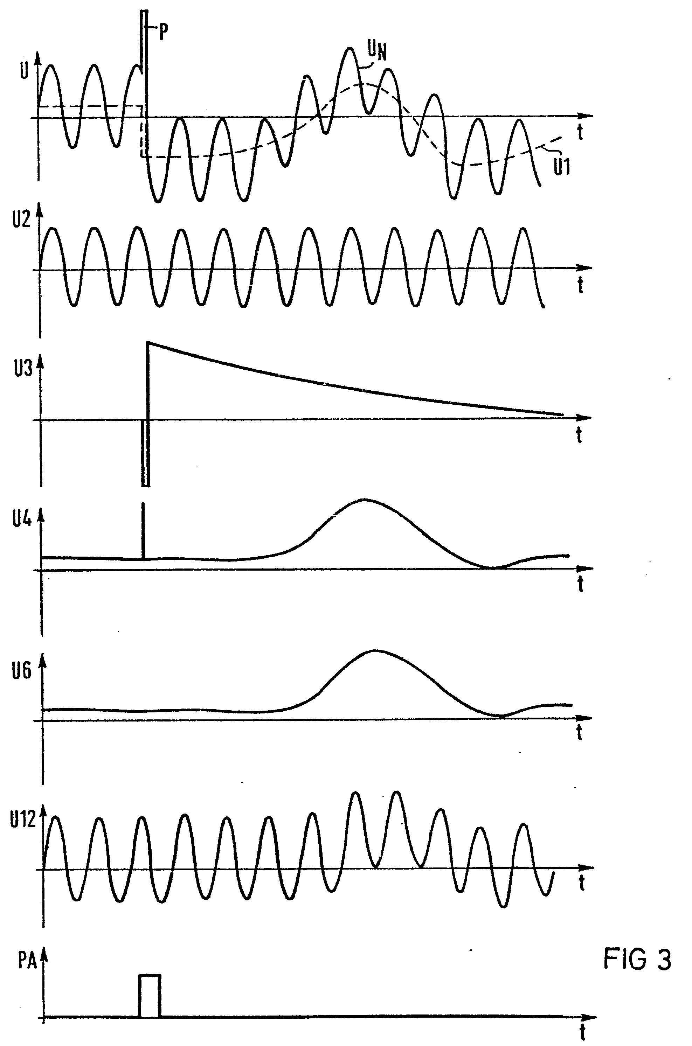

Dieser Fall wird anhand der Fig. 2 in Verbindung mit den Spannungsverläufen der Fig. 3 erläutert. Die Eingangsspannung U besteht demnach aus dem EKG-Verlauf U1, dem die Netzspannung $U_N$ und ein Schrittmacherimpuls P überlagert sind. Solange noch kein Stimulationsimpuls P auftritt, entspricht die Signalverarbeitung dem Fall b). Fällt ein Stimulationsimpuls P an, so verläuft die Austastung entsprechend Fall c), wobei jedoch hinsichtlich des Verhaltens des selektiven Netzfrequenzfilters B1 eine Besonderheit zu beachten ist: Zu Beginn der Austastung wird nämlich auch bei nunmehr geöffnetem Schalter S1 das selektive Netzfrequenzfilter nicht einfach abgeschaltet; das Filter B1 schwingt vielmehr auf der vorhergehend noch vorhandenen Netzfrequenz weiter. Auf diese Weise wird also am Ausgang eine Ausgangsspannung U2 simuliert, die der Netzfrequenz nach Amplitude und Phase gleich ist. Dieser Vorgang der Selbsterregung wird während der Zeit der Austastung, d. h. solange auch der Schalter S2 geschlossen ist, über die Rückschleife B1, U2, A3, U10, S2, U12, B1 aufrechterhalten. Das Spannungssignal U12 am Eingang des selektiven Netzfrequenzfilters B1 folgt demnach also dem in Fig. 3 dargestellten zeitlichen Verlauf. Die Phase der Austastung eines Schrittmacherimpulses ist dabei mit PA(t) aufgezeichnet. Während der Phase der Austastung wird also in der Mischstufe A1 durch das weiter hinzugeführte simulierte Signal U2 die auch weiterhin vorhandene Netzspannung $U_N$ im Eingangssignal U kompensiert. Die Ausgangsspannung U4 der Mischstufe A1 bleibt also frei vom Netzbrumm.

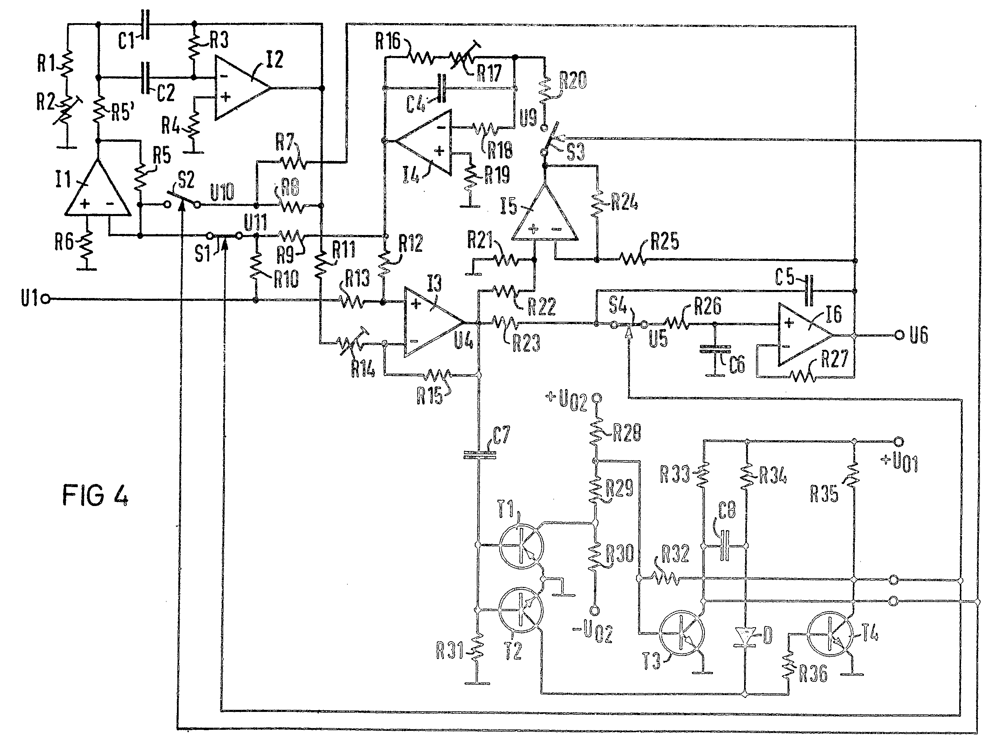

Die Fig. 4 zeigt eine Schaltungsanordnung gemäß dem Prinzipschaltbild der Fig. 2 in ausführlicher Darstellung. Dabei besteht folgender Zusammenhang mit dem Blockschaltbild der Fig. 2:

A1:     R11, R12, R13, R14, R15, I3
A2:     R9, R10
A3:     R7, R8
A4, B4: R21, R22, R24, R25, I5
B1:     R1, R2, R3, R4, R5, R6, C1, C2, I1, I2
B2:     R28, R29, R30, R31, R32, R33, R34, R35,
        R36, C7, C8, T1, T2, T3, T4, D1
B3:     R23, R26, R27, C5, C6, I6
B5:     R16, R17, R18, R19, C4, I4

wobei die Bauelemente R jeweils Ohm'sche Widerstände, die Bauelemente C Kondensatoren, die Bauelemente T Transistoren und die Bauelemente D Dioden sind. Bei den Bauelementen I handelt es sich um Operationsverstärker und bei den Bauelementen S um Analogschalter. Im speziellen dient im Netzfrequenzfilter B1 der Trimmwiderstand R2 zum Abgleich der Frequenz des Netzfrequenzfilters auf 50 Hz, R14 in der Mischstufe A1 ist hingegen für den Amplitudenabgleich vorgesehen, so daß in Überlagerung mit dem ebenfalls Störbrumm umfassenden Eingangssignal U der Störbrumm kompensiert wird. Mit R17 im Zeitkonstanten-Glied B5 wird das RC-Glied C4, R16 + R17 auf die Zeitkonstante des Überschwingers des Herzschrittmacherimpulses eingestellt.

Wie aus dem Schaltbild ferner zu ersehen ist, bestehen die Mischstufen A1 bis A4 aus Widerstandsnetzwerken, die im Zusammenspiel mit Operationsverstärkern lineare Spannungsüberlagerung gestatten. Das selektive Netzfrequenzfilter B1 ist ein aktives Filter mit vorgeschaltetem Inverter I1. Das Filter ist in der Dimensionierung so ausgelegt, daß bei geschlossenem Schalter S2 ein Aktivfilter entsteht, das nahe dem Übergang von gedämpftem zu ungedämpftem Zustand betrieben ist. Beim Abschalten des Filters von der Netzstromkomponente $U_N$ schwingt dieses Filter auf der Netzfrequenz mit der vorhergehend abgeschalteten Amplitude der Netzspannung weiter. Der Schaltungsteil zur Unterdrückung des Herzschrittmacherimpulses arbeitet nach einem Verfahren ähnlich dem der Schaltungsanrodnung der GB-PS 1 450 081. Die Erkennung des Schrittmacherimpulses erfolgt demnach in bekannter Weise entsprechend aus Steilheit und Amplitude des Eingangssignals. Als besonderer Vorteil kommt jedoch jetzt hinzu, daß die Erkennung des Pacemakerimpulses erst nach Unterdrückung der Netzfrequenz erfolgt, so daß große Netzfrequenzamplituden nicht fälschlich als Schrittmacherimpuls erkannt werden. Das RC-Glied R34, C8 legt die Dauer der Phase einer Austastung eines Schrittmacherimpulses fest. Die Dauer ist auf etwa t2 = 9 ms dimensioniert. Zur Speicherung des der Austastung vorhergehenden Spannungswertes dient der Kondensator C6.

Mit der Schaltungsvorrichtung der Fig. 2 und 4 wird bevorzugt die Grundwelle der Netzfrequenz selektiert und kompensiert. Es ist selbstverständlich, daß durch Parallelschaltung mehrerer entsprechend unterschiedlich selektiver Filter auch beliebig zusätzlich Oberwellen der Netzfrequenz unterdrückt werden können. Das Zeitkonstanten-Glied B5 ist in den Schaltungsanordnungen der Fig. 2 und 4 speziell auf eine eingliedrige e-Funktion ausgelegt. Es entspricht ebenfalls der Selbstverständlichkeit, daß auch Zeitkonstanten-Glieder mit den einer e-Funktion abweichendem Zeitverhalten Einsatz finden können, beispielsweise mit einer solchen Zeitfunktion, die die Verfälschung der e-Funktion des Überschwingers des Herzschrittmacherimpulses speziell durch die Zeitkonstante eines EKG-Vorverstärkers bei der Kompensation berücksichtigt. Alle diese Modifikationen fallen selbstverständlich unter der Erfindung.

## Patentansprüche

1. Verfahren zur Unterdrückung von Störsignalen in einem Nutzsignal, wobei die Störsignale als Gemisch aus schmalen Störimpulsspitzen hoher Amplitude und Störbrumm anfallen können, und wobei mittels Störspitzen- und Störbrumm-Erkennungseinrichtungen das Vorhandensein von Störimpulsspitzen und/oder Störbrumm separat erkannt wird und mit dem Erkennen Unterdrückereinrichtungen zur Unterdrückung der Störimpulsspitzen und/oder des Störbrumms im Nutzsignal aktiviert werden und wobei der Störbrumm bei Vorhandensein speziell durch ein Störbrummfilter erkannt und durch gegensinnige Überlagerung mit dem gestörten Nutzsignal vom Nutzsignal eliminiert wird, dadurch gekennzeichnet, daß ein anfallendes Nutzsignal (U1) mit möglicherweise überlagerten Störsignalen ($U_N$ und/oder P) nur so lange einem speziell auch auf Eigenschwingbetrieb umschaltbaren Störbrummfilter (B1) zwecks Herausfilterung speziell des Störbrumms ($U_N$) zugeführt wird, wie von der Erkennungseinrichtung (B2) für schmale Störimpulsspitzen (P) hoher Amplitude keine solche Störimpulsspitzen erkannt werden, und daß auch nur in diesem Falle die herausgefilterte Störbrummkomponente (U2) im Originalverlauf dem Nutzsignal (U1) im Meßsignalweg vor der Erkennungseinrichtung (B2) für Störimpulsspitzen (P) zwecks Kompensation des diesem überlagerten Störbrumms ($U_N$) gegensinnig überlagert wird, daß jedoch in dem Falle, daß von der Erkennungseinrichtung (B2) eine Störimpulsspitze (P) erkannt wird, das Störbrummfilter (B1) vom Eingangssignal abgeschaltet und gleichzeitig auf Eigenschwingbetrieb umgeschaltet wird, so daß es mit der Amplitude und Frequenz der vorhergehend selektierten Störbrummkomponente ohne Phasensprung weiterschwingt, wobei als Anfangswert für die Eigenschwingung des Störbrummfilters (B1) jener Signalwert dient, der im Augenblick des Erkennens einer Störimpulsspitze (P) der unmittelbar vorhergehende Signalwert der Eingangsspannung (U) ist und der einerseits in einem ausgangsseitigen Speicher (B3) als ausgangsseitiges Nutzsignal für die Zeitdauer einer Austastung der Störimpulsspitze gespeichert wird und am Ende der Austastung den Anfangswert für die Fortsetzung des Signalspannungsverlaufes (U6) bildet und der andererseits zur Bildung des Anfangswertes für die Eigenschwingung auch zum Eingang des Störbrummfilters (B1) rückgeführt wird, und daß die vom Störbrummfilter (B1) so künstlich erzeugte Schwingung schließlich wenigstens während der Zeitdauer der Unterdrückung der Störimpulsspitze (P) dem Nutzsignal im Meßsignalweg vor der Erkennungseinrichtung (B2) zur Kompensation des Störbrumms überlagert wird, so daß der Erkennungseinrichtung (B2) immer von Störbrumm ($U_N$) bereinigtes Nutzsignal (U1 = U4) angeboten wird.

2. Verfahren nach Anspruch 1, dadurch gekennzeichnet, daß der Signalwert der Eingangsspannung (U), der dem Augenblick des Erkennens einer Störimpulsspitze (P) unmittelbar vorhergeht, auf Grund Signalverzögerung vor Abspeicherung aus dem Meßsignal gewonnen wird.

3. Verfahren nach Anspruch 1 oder 2, dadurch gekennzeichnet, daß während der Zeitdauer der Austastung einer Störimpulsspitze (P) in einem Zeitkonstantenglied (B5) ein Spannungszeitverlauf (U3) entsprechend jenem des Überschwingers einer Störimpulsspitze erzeugt wird, der durch gegensinnige Überlagerung mit dem Eingangssignal den Überschwinger der Störimpulsspitze im Nutzsignal (U1) kompensiert.

4. Verfahren nach Anspruch 3, dadurch gekennzeichnet, daß das Zeitkonstantenglied (B5) einen Spannungszeitverlauf erzeugt, der von vornherein dem Originalzeitverlauf des Überschwingers im Nutzsignal entgegengesetzt polarisiert ist.

5. Verfahren nach Anspruch 3 oder 4, dadurch gekennzeichnet, daß nach Erkennen einer Störimpulsspitze (P) zu Beginn der Austastung der in diesem Augenblick anstehende Spannungswert, der dem Spitzenwert des Überschwingers entspricht, vom Zeitkonstantenglied (B5) während der Austastdauer gehalten und erst mit Ende der Austastung dem abklingenden Überschwinger ein entsprechend abklingender Spannungszeitverlauf des Zeitkonstantengliedes im Sinne der Kompensation überlagert wird.

6. Verfahren nach einem der Ansprüche 3 bis 5, dadurch gekennzeichnet, daß der Spannungszeitverlauf (U3) am Ausgang des Zeitkonstantengliedes (B5) auch dem Eingangssignal (U) vor Zuführung zum Eingang des Störbrummfilters (B1) im Sinne der Kompensation beigemischt wird.

7. Schaltungsanordnung zur Durchführung des Verfahrens nach einem der Ansprüche 1 bis 6, mit einer Störspitzen-Erkennungsrichtung (B2) und einer davon gesteuerten Austasteinrichtung (S4) für Störimpulsspitzen (P) sowie einem Störbrummfilter (B1) mit einem Eingang für das möglicherweise mit Störsignalen ($U_N$ und/ oder P) überlagerte Nutzsignal (U1) und einem Ausgang, der zum Zwecke der gegensinnigen Überlagerung des Nutzsignals samt Störsignal mit dem vom Filter (B1) ausgefilterten Störbrumm (U2) am Meßsignalweg angeschaltet ist, dadurch gekennzeichnet, daß der Eingang des Störbrummfilters (B1) über einen Ab- oder Umschalter (S1) vom Eingangssignal abschaltbar ist, welcher Schalter (S1) von einem Schaltausgang der Erkennungseinrichtung (B2) für Störimpulsspitzen (P) bei Auftreten einer solchen Spitze aktivierbar ist, daß das Störbrummfilter (B1) einen Rückkreis aufweist, der einen Rückkoppelschalter (S2) umfaßt, der gegensinnig zum Ab- oder Umschalter (S1) geschaltet wird, und der auch eine Mischstufe (A3) beinhaltet, der das Ausgangssignal (U2) des Störbrummfilters (B1) und ein Spannungswert (U6) zuführbar sind, der einem gespeicherten Ausgangsspannungswert des Nutzsignals entspricht, der im Augenblick des Erkennens einer Störimpulsspitze durch die Störimpulsspitzenerkennungseinrichtung (B2) in einer Speichereinheit (B3) gespeichert wurde, und daß der Erkennungseinrichtung (B2) für eine Störimpulsspitze eine Mischstufe (A1) für ein Nutzsignal mit möglicherweise überlagertem Störbrumm und überlagerter Störimpulsspitze und für das Ausgangssignal (U2) des Störbrummfilters (B1) vorgeschaltet ist.

8. Schaltungsanordnung nach Anspruch 7, dadurch gekennzeichnet, daß das Störbrummfilter (B1) ein Aktivfilter ist, das durch Schließen eines Rückkoppelschalters (S2) beim Öffnen des eingangsseitigen Ab- oder Umschalters (S1) nahe dem Übergang von gedämpftem zu ungedämpftem Zustand betrieben ist.

9. Schaltungsanordnung nach Anspruch 7 oder 8, dadurch gekennzeichnet, daß dem Ab- oder Umschalter (S1) am Eingang des Störbrummfilters (B1) eine Mischstufe (A2) für das mit Störbrumm behaftete Nutzsignal und ein von einem Zeitkonstanten-Glied (B5) erzeugtes Kompensationssignal (U3) für den Überschwinger einer Störimpulsspitze vorgeschaltet ist.

10. Schaltungsanordnung nach Anspruch 9, dadurch gekennzeichnet, daß die Störimpulsspitzen-Erkennungseinrichtung (B2) mit dem Erkennen einer Störimpulsspitze mittels Schalter (S3) das Zeitkonstanten-Glied (B5) an einen Komparator (B4) anschaltet, der dem Zeitkonstanten-Glied (B5) den Spitzenwert des Überschwingers der Störamplitudenspitze zur raschen Aufladung auf diesen Wert mitteilt.

11. Schaltungsanordnung nach Anspruch 10, dadurch gekennzeichnet, daß zum Erhalt einer Regelschleife der Ausgang (U3) des Zeitkonstantengliedes (B5) an einen weiteren Eingang der Mischstufe (A1) angeschlossen und dem Komparator (B4) eingangsseitig eine Mischstufe (A4) mit zwei Eingängen vorgeschaltet ist, deren einer Eingang mit dem Ausgang (U4) der Mischstufe (A1) und deren zweiter Eingang mit dem Ausgang (U6) der Speichereinheit (B3) verbunden ist, der das vom Störbrumm bereinigte Eingangssignal eingangsmäßig zugeleitet ist.

## Claims

1. Process for suppressing interference signals in a usefal signal, wherein the interference signals can occur as a mixture of narrow interference pulse peaks of high amplitude and interference hum and wherein interference peak recognition and interference hum recognition devices are used to separately recognise the presence of interference pulse peaks and/or interference hum and upon recognition, suppression devices are activated to suppress the interference pulse peaks and/or the interference hum in the useful signal, and wherein, in the event that it is present, the interference hum is specially recognised by an interference hum filter and is eliminated from the useful signal by opposing superimposition on the disturbed useful signal, characterised in that when a useful signal (U1) occurs, which is possibly superimposed by interference signals (U_N and/or P), it is specially conducted to an interference hum filter (B1), which can be switched over to natural oscillation operation, for the purpose of specifically filtering out the interference hum (U_N) only for such time as the recognition device (B2) for narrow interference pulse peaks (P) of high amplitude recognises no such interference pulse peaks, and that also only under these condition the filtered out interference hum component (U2) in the original flow is superimposed in opposing fashion onto the useful signal (U1) in the measured signal path prior to the recognition divice (B2) of interference pulse peaks (P) in order to compensate the superimposed interference hum (U_N), but in the event that the recognition device (B2) does recognise an an interference pulse peak (P), the interference hum filter (B1) is switches off by the input signal and at the same time a switch-over is made to natural oscillation operation, so that it continues to oscillate at the amplitude and frequency of the previously selected interference hum component without any phase jump, where the starting value for the natural oscillation of the interference hum filter (B1) is represented by the signal value of the input voltage (U), and which on the ohne hand is stored in a store (B3) for the duration of the blanking of the interference pulse peak and therefore, at the end of the blanking, forms the starting value for the continuation of the signal voltage course (U6) and which on the other hand is returned to the input of the interference hum filter (B1) in order to form the starting value for the natural oscillation and that the oscillation which in this way is artificially produced by the interference hum filter (B1) is finally at least during the time of the suppression of the interference pulse peak (P), superimposed upon the useful signal in the measured signal path prior to the recognition device (B2) in order to compensate the interference hum, so that the recognition device (B2) is always supplied with a useful signal (U1 = U4) which has interference hum (U_N) removed.

2. Process as claimed in Claim 1, characterised in that the signal value of the input voltage (U) which directly precedes the instant of recognition of an interference pulse peak (P) is acquired from the measured signal as a result of signal delay prior to storage.

3. Process as claimed in Claim 1 or 2, characterised in that during the time of the blanking of an interference pulse peak (P) in a time constant element (B5), a voltage time curve (U3) is produced in accordance with that of the overshoot of an interference pulse peak and, by opposing superimposition upon the input signal, this curve compensates the overshoot of the interference pulse peak in the useful signal (U1).

4. Process as claimed in Claim 3, characterised in that the time constant element (B5) produces a voltage time curve which from the start is polarised in opposition to the original time curve of the overshoot in the useful signal.

5. Process as claimed in Claim 3 or 4, characterised in that following the recognition of an interference pulse peak (P) at the beginning of the blanking, the voltage value which occurs at the peak value of the overshoot, is maintained by the time constant element (B5) during the blanking time and only at the end of the blanking is the dying overshoot superimposed by a correspondingly dying voltage time curve of the time constant element in the sense of compensation.

6. Process as claimed in one of the Claims 3 to 5, characterised in that the voltage time curve (U3) at the output of the time constant element (B5) is also mixed with the input signal (U) for compensation prior to supply to the input of the interference hum filter (B1).

7. Circuit arrangement for the implementation of the process claimed in one of the Claims 1 to 6, with an interference peak recognition device (B2) and, controlled by the latter, a blanking devise (S4) for interference pulse peaks (P) and with an interference hum filter (B1) having an input for the useful signal (U1) which may possibly be superimposed by interference signals (U_N and/or P) and having an output which, for the purpose of the oppositely directed superimposition of the useful signal, including the interference signal, with the interference hum (U2) filtered out by the filter (B1), is connected to the measured signal path, characterised in that the input of the interference hum filter (B1) can be disconnected from the input signal via a disconnecting switch or change-over switch (S1), which can be activated by a switching output of the recognition device (B2) for interference pulse peaks (P) on the occurrence of such a peak and that the interference hum filter (B1) possesses a return circuit which comprises a feedback switch (S2), which is switched in the opposite direction to the disconnecting switch or change-over switch (S1), and which also contains a mixer stage (A3) which can be supplied with the output signal (U2) of the interference hum filter (B1) and with a voltage value (U6) which corresponds to a stored output voltage value of the useful signal which, at the instant of the recognition of an interference pulse peak by the interference pulse peak recognition device (B2) has been stored in a storage unit (B3), and that the recognition device (B2) for an interference pulse peak is preceded by a mixer stage (A1) for a useful signal possibly superimposed by an interference hum and superimposed by an interference pulse peak and for the output signal (U2) of the interference hum filter (B1).

8. Circuit arrangement as claimed in Claim 7, characterised in that the interference hum filter (B1) is an active filter which, as a result of the closure of a feedback switch (S2) at the time of the opening of the input-end disconnecting switch (S1) is operated close to the transition from the attenuated to the unattenuated state.

9. Circuit arrangement as claimed in Claim 7 or 8, characterised in that the disconnecting switch or changeover switch (S1) at the input of the interference hum filter (B1) is preceded by a mixer stage (A2) for the useful signal which is subject to interference hum, and a compensation signal (U3), produced by a time constant element (B5), for the overshoot of an interference pulse peak.

10. Circuit arrangement as claimed in Claim 9, characterised in that on the recognition of an interference pulse peak, by means of switches (S3) the interference pulse peak recognition device (B2) connects the time constant element (B5) to a comparator (B4) which informs the time constant element (B5) of the peak value of the overshoot of the interference amplitude peak for the purpose of rapid charging to this value.

11. Circuit arrangement as claimed in Claim 10, characterised in that in order that a regulating loop may be formed, the output (U3) of the time constant element (B5) is connected to a further input of the mixer stage (A1), and the input of the comparator (B4) is preceded by a mixer stage (A4) having two inputs, one of which is connected to the output (U4) of the mixer stage (A1) and the second of which is connected to the output (U6) of the storage unit (B3) which is supplied at its input with the input signal which has the interference hum removed.

## Revendications

1. Procédé pour supprimer des signaux parasites dans un signal utile, les signaux parasites pouvant se présenter sous la forme d'un mélange d'étroites pointes d'impulsions parasites de grande amplitude avec des ronflements parasites, du type dans lequel on peut, à l'aide du dispositif de reconnaissance de pointes parasites et de ronflements parasites, reconnaître séparément la présence de pointes d'impulsions parasites et/ou de ronflements parasites et on peut activer des dispositifs de suppression pour supprimer les pointes d'impulsions parasites et/ou le ronflement parasite dans le signal utile, dès ladite reconnaissance alors que le ronflement parasite, lorsqu'il est présent, est reconnu particulièrement à l'aide d'un filtre pour le ronflement parasite et est éliminé du signal utile par superposition, en sens inverse, avec le signal utile perturbé, caractérisé par le fait qu'un signal utile (U1) qui se présente avec éventuellement des signaux parasites superposés ($U_N$ et/ou P) n'est appliqué, pour la séparation particulière du ronflement parasite ($U_N$), à un filtre de ronflement parasite (B1) particulier et également susceptible d'être commuté pour un fonctionnement en auto-oscillation que pendant la durée pendant laquelle le dispositif de reconnaissance (B2) pour des pointes étroites d'impulsions parasites (P) de forte amplitude ne reconnait pas de telles pointes d'impulsions parasites et que seulement dans ce cas la composante filtrée (U2) de ronflement parasite dans l'allure originel est superposée en sens inverse au signal utile (U1) dans la voie du signal de musere située en avant du dispositif de reconnaissance (B2) pour des pointes d'impulsions parasites (P) en vue de compenser le ronflement parasite ($U_N$) superposé au signal utile (U1), mais que dans le cas où le dispositif de reconnaissance (B2) reconnait une pointe d'impulsion parasite (P) le filtre de ronflement parasite (B1) est débranché du signal d'entrée et est en même temps commuté en fonctionnement d'auto-oscillation, en sorte qu'il continue à osciller, sans changement brusque de la phase, avec l'amplitude et la fréquence de la composante de ronflement parasite sélectionnée antérieurment, la valeur du signal qui sert de valeur initiale pour l'oscillation propre du filtre de ronflement parasite (B1) étant la valeur du signal de la tension d'entrée (U) immédiatement antérieurepar rapport à l'instant de la reconnaissance d'une pointe d'impulsion parasite (P) et qui est, d'une partmémorisée dans une mémoire (B3) pour la durée d'une suppression de la pointe d'impulsion parasite et forme de ce fait, à la fin de la suppression, la valeur initiale pour la suite de l'allure de la tension du signal (U6) et qui, d'autre part, est renvoyée également à l'entrée du filtre de ronflement parasite (B1) pour former la valeur initiale pour l'oscillation propre, et que l'oscillation ainsi artificiellement produite par le filtre de ronflement parasite (B1) est finalement superposée, au moins pour la durée de la suppression de la pointe d'impulsion parasite (P), au signal utile, dans la voie du signal de mesure, en avant du dispositif de reconnaissance, en vue de compenser le ronflement parasite, en sorte que le dispositif de reconnaissance (B2) ne reçoit qu'un signal utile (U1 = U4) qui est toujours dépourvu du ronflement parasite ($U_N$).

2. Procédé selon la revendication 1, caractérisé par le fait que la valeur du signal de la tension d'entrée (U) qui précéde immédiatement l'instant auquel est reconnue une pointe d'impulsion parasite (P) est obtenue à partir du signal de mesure, sur la base du retard du signal avant mémorisation.

3. Procédé selon la revendication 1 ou 2, caractérisé par le fait que pendant la durée de la suppression d'une pointe d'impulsion parasite P on produit dans un dispositif à constante de temps (B5), une courbe caractéristique tension/temps (U3) qui correspond à celle de la suroscillation d'une pointe d'impulsion parasite et qui compense, par superposition en sens opposé avec le signal d'entrée, la suroscillation de la pointe d'impulsion parasite dans le signal utile (U1).

4. Procédé selon la revendication 3, caractérisé par le fait que le dispositif à constante de temps (B5) fournit une courbe caractéristique tension/temps qui, à l'avance, est polarisée en sens inverse par rapport à l'allure du temps originel de la suroscillation dans le signal utile.

5. Procédé selon la revendication 3 ou 4, caractérisé par le fait qu'aprés la reconnaissance d'une pointe d'impulsion parasite (P), au début de la suppression, la valeur de la tension présente à cet instant et correspondant à la valeur de pointe de l'oscillation de dépassement est conservée par le dispositif à constante de temps (B5) pendant la durée de la suppression et ce n'est qu'à la fin de la suppression à l'oscillation de dépassement qui est superposée, dans le sens d'une compensation, à l'oscillation de dépassement que décroit une allure tension/temps, décroissant de façon correspondante, du dispositif à constante de temps.

6. Procédé selon l'une des revendications 3 à 5, caractérisé par le fait que la courbe caractéristique de la tension par rapport au temps (U3) est mélangée à la sortie du dispositif à constante de temps (B5), et dans le sens de la compensation, également au signal déntrée (U), avant son application à l'entrée du filtre de ronflement parasite (B1).

7. Montage pour la mise en oeuvre du procédé selon l'une des revendications 1 à 6, comprenant un dispositif de reconnaissance (B2) de pointes de parasites et un dispositif de suppression (S4) pour les pointes des impulsions parasites (P) contrôlé par ledit dispositif de reconnaissance, ainsi qu'un filtre de ronflements parasites (B1) comportant une entrée pour le signal utile (U1) auquel sont éventuellement superposés des signaux parasites ($U_N$ et/ou P) et une sortie qui, en vue d'une superposition en sens inverse du signal utile, comprenant le signal parasite, avec le ronflement parasite (U2) filtrée par le filtre (B1), est reliée à la voie du signal de mesure, caractérisé par le fait que l'entrée du filtre pour le ronflement parasite (B1) est susceptible d'être débranché du signal d'entrée à l'aide d'un interrupteur ou commutateur (S1) susceptible d'être activé par une sortie de commutation du dispositif de reconnaissance (B2) des pointes d'impulsions parasites (P) à l'apparition d'une telle pointe, que le filtre de ronflement parasite (B1) comporte un circuit de réaction comprenant un commutateur de réaction (S2) qui est commandé en sens inverse de l'interrupteur ou commutateur (S1) ainsi qu'un étage mélangeur (A3) auquel peuvent être appliqués le signal de sortie (U2) du filtre du ronflement parasite (B1) et une valeur de tension (U6) qui correspond à une valeur de tension initiale, mémorisée, du signal utile qui, à l'instant de la reconnaissance d'une pointe d'impulsion parasite par le dispositif de reconnaissance (B2) des pointes d'impulsions parasites, a été mémorisé dans une unité de mémoire (B3), et qu'en amont du dispositif de reconnaissance (B2) pour une pointe d'impulsion parasite est prévu un étage mélangeur (A1) pour un signal utile auquel sont éventuellement superposés un ronflement parasite et une pointe d'impulsion parasite et pour le signal de sotie (U2) du filtre du ronflement parasite (B1).

8. Montage selon la revendication 7, caractérisé par le fait que le filtre du ronflement parasite (B1) est un filtre actif qui, par la fermeture d'un commutateur de réaction (S2) et à l'ouverture de l'interrupteur ou commutateur (S1) situé de côté entrée, opère près du passage, de l'état amorti à l'état non amorti.

9. Montage selon la revendication 7 ou 8, caractérisé par le fait qu'à l'entrée du filtre pour le ronflement parasite (B1), il est prévu en amont de l'interrupteur ou commutateur (S1) un étage mélangeur (A2) pour le signal utile affecté d'un ronflement parasite et un signal (U3) produit par un dispositif à constante de temps (B5), 5), pour compenser l'oscillation de dépassement d'une pointe d'impulsion parasite.

10. Montage selon la revendication 9, caractérisé par le fait que le dispositif de reconnaissance (B2) des pointes d'impulsions parasites relie, lors de la reconnaissance d'une pointe d'impulsion parasite, et à l'aide d'un commutateur (S3), le dispositif à constante de temps (B5) avec un comparateur (B4) qui communique au dispositif à constante de temps (B5) la valeur de crête de l'oscillation de dépassement de la pointe d'amplitude parasite pour la charge à cette valeur.

11. Montage selon la revendication 10, caractérisé par le fait que pour obtenir une boucle de régulation, la sortie (U3) du dispositif à constante de temps (B5) est relié à une autre entrée de l'étage mélangeur (A1) et qu'en amont du comparateur (B4) est prévu, côté entrée, un étage mélangeur (A4) à deux entrées dont l'une est reliée avec la sortie (U4) de l'étage mélangeur (A1) et la seconde avec la sortie (U6) de l'unité de mémoire (B3) à laquelle on applique à l'entrée le signal d'entrée débarrassé du ronflement parasite.

0 003 588

FIG 1

FIG 2

13

FIG 3

FIG 4